# EUROPÄISCHE PATENTANMELDUNG

(11) **EP 0 955 541 A2**
(43) Veröffentlichungstag der Anmeldung: **10.11.1999**
(21) Anmeldenummer: 99109017.6
(22) Anmeldetag: 07.05.1999
(51) Int. Cl.: G01N 27/42

(54) **Verfahren und Messzelle zum Messen des Feuchtegehaltes von Gasen**

(30) Priorität: 08.05.1998 DE 29808249 U; 10.04.1999 DE 29906390 U
(71) Anmelder: Gerhard, Klaus, 63825 Blankenbach (DE)
(72) Erfinder: Gerhard, Klaus, 63825 Blankenbach (DE)
(74) Vertreter: Fuchs Mehler Weiss & Fritzsche

(57) **Zusammenfassung**

Die Erfindung betrifft ein Meßverfahren und eine Meßzelle für ein Gerät zum Messen des Feuchtegehalts Meßzelle für ein Gerät zum Messen des Feuchtegehaltes von Gasen mit einem von dem zu messenden Gas durchströmbaren Gehäuse (1) mit Einlaß- und Auslaßmitteln (2,3) für das Meßgas und einer in dem Gehäuse (1) vorgesehenen Meßfühleranordnung 4 mit mindestens einem Meßfühler (5,6) mit einem Isolatorkörper, der an seiner Oberfläche mit einem hykroskopischen Elektrolytfilm versehen ist, und mindestens einer Elektrodenwicklung (8,9) mit einem Paar um den Isolatorkörper (7) gewickelter und in den Elektrolytfilm eingebetteter, beabstandeter Elektrodendrähte, die mit durch das Gehäuse (1) nach außen geführten elektrischen Anschlüssen (10,11,12) versehen sind, für das Anlegen einer Meßspannung an die Elektrodenwicklungen (8,9).

Die Meßzelle ist erfindungsgemäß dadurch gekennzeichnet, daß zwei Elektrodenwicklungen (8,9) vorgesehen sind, die über ihre elektrischen Anschlüsse (10,11,12) unabhängig voneinander mit einer Meßspannungsquelle verbindbar sind.

## Beschreibung

Die Erfindung betrifft ein Verfahren zum Messen des Feuchtegehaltes von Gasen, bei dem ein definierter Strom des zu messenden Gases einen Meßfühler mit einem Paar in einen hygroskopischen Elektrolytfilm eingebetteter und mit einer Meßspannung beaufschlagter Elektroden umströmt, und der zwischen den Elektroden fließende Elektrolysestrom als Maß für den Feuchtegehalt des Gases bestimmt wird.

Die Erfindung betrifft ferner ein Gerät zum Durchführen einer solchen Messung und eine Meßzelle für ein solches Gerät.

In verschiedenen Industriezweigen, wie bei der Gasherstellung und bei Kältemittelherstellern, um nur einige zu nennen, kommen Gase zum Einsatz, die typischerweise einen Restfeuchtegehalt aufweisen, den es zu bestimmen gilt, um ggf. weitere Maßnahmen einleiten zu können, wie Einschaltung einer Trocknerstufe oder dergleichen, wenn der Restfeuchtegehalt einen bestimmten Betrag übersteigt. Das gleiche gilt für Industrien, bei denen die Luftfeuchte oder die Feuchte in anderen Gasen einen bestimmten Betrag nicht überschreiten darf.

Es ist neben kapazitiven Feuchtemessmethoden ein auf einem chemischen Meßprinzip beruhendes Verfahren zur Messung des Restfeuchtegehaltes in Gasen bekannt, sowie ein nach diesem Verfahren arbeitendes Gerät, das eine Meßzelle besitzt, die einen Träger aus isolierendem Material mit einem Elektrolytfilm aufweist, in welchem zwei parallel um den Träger gewickelte Platinenelektroden eingebettet sind, und die einen Einlaß und einen Auslaß für das Meßgas aufweist. Trifft nun das einströmende Meßgas in der Meßzelle auf den Elektrolytfilm, so wird die im Meßgas vorhandene Feuchte von dem Elektrolyten teilweise absorbiert und an den Platinelektroden aufgrund einer an diese angelegten Gleichspannung in Wasserstoff und Sauerstoff durch Elektrolyse zerlegt. Die Stromstärke, die sich bei der Elektrolyse des absorbierten Wasserdampfes bei einer bestimmten, angelegten Spannung ergibt, dient als Maß für die Feuchtekonzentration im Meßgas. Dabei muß eine konstante Strömungsmenge an Meßgas eingehalten werden, da die vorstehende Messung in ihrer Genauigkeit strömungsabhängig ist.

Das bekannte Gerät, das auch als Elektrolyse-Hygrometer bezeichnet wird, erlaubt eine Messung der Feuchte in allen neutralen und sauren Gasen; es ist dabei ein besonders robustes Gerät, welches auch in extrem korrosiven Medien eingesetzt werden kann. Das besonders unempfindliche chemische Meßprinzip garantiert jahrelangen Betrieb ohne großen Wartungs- bzw. Kostenaufwand.

Es hat sich aber gezeigt, daß das bekannte Gerät bei längerem Betrieb plötzlich auftretende Feuchtespitzen gelegentlich nicht mehr deutlich erkennen kann. Ferner ist die prozentuale Umsetzungsmenge an meßwirksamer Feuchte beschränkt. Sie liegt bei etwa 30%. Dadurch ist auch der Anfangsabschnitt des Meßbereichs (geringer Feuchtegehalt) nicht ideal linear ausgebildet.

Der Erfindung liegt die Aufgabe zugrunde, das eingangs bezeichnete Meßverfahren bzw. das zugehörige Gerät zur Messung des Feuchtegehalts in Gasen so zu gestalten, daß es auch bei längerem Betrieb plötzlich auftretende Feuchtespitzen deutlich erkennen kann.

Diese Aufgabe wird verfahrensmäßig erfindungsgemäß durch die kennzeichnenden Merkmale des Patentanspruches 1 gelöst.

Gemäß der neuen Verfahrensweise wird bei merkbarer gewisser Erschöpfung des Elektrolyten zwischen den Elektroden eines Meßfühlers auf einen anderen Meßfühler umgeschaltet. Vorzugsweise werden zwei Meßfühler verwendet, die mit bestimmten Zeitintervallen wechselweise betrieben werden. Der Umschaltzyklus kann beispielsweise zwischen etwa 1 und 10 min. betragen. Bei einem entsprechenden Gerät ist an der mit den Meßfühlern elektrisch zu verbindenden Versorgungs- und Erfassungseinheit die Intervallzeit der beiden Meßfühler vorzugsweise frei wählbar. In speziell bevorzugter Ausführungsform des Verfahrens wird der letzte Meßwert des abgeschalteten Meßfühlers gespeichert und mit dem Meßwert des zugeschalteten, aktuellen Meßfühlers verglichen. Werden hierbei Abweichungen festgestellt, die eine gewisse zulässige Differenz von beispielsweise 1 % überschreiten, kann eine Alarmauslösung vorgesehen sein. Wird eine solche Differenz nicht überschritten und zeigt der aktuelle Meßfühler nach seinem Zuschalten eine schnelle Reaktion in seinem Verhalten, z.B. einen bestimmten Differentialquotienten der Meßwertänderung, wird der gespeicherte Meßwert des abgeschalteten Sensors aufgegeben.

Es kann auch sinnvoll sein, beim Wechsel der Meßfühler beide Fühler für eine begrenzte Zeit überlappend zu betreiben und dabei ihr Verhalten zu vergleichen.

Es sei ausdrücklich darauf hingewiesen, daß verläßliche Meßwerte im wesentlichen nur dann erhalten werden, wenn die zwei oder auch mehreren Meßfühler mit parallelen Strömen des zu messenden Gases betrieben werden. Bei Betrieb mit zwei wechselweise zu betreibenden Meßfühlern wird zweckmäßigerweise ein zur Verfügung stehender definierter Meßgasstrom in zwei gleiche Teilströme aufgeteilt, von denen jeder ständig einem der Meßfühler zugeführt wird. Der nicht aktuelle Meßfühler soll gerade auch während seiner Ruhezeit zur Regeneration ständig vom Meßgas umspült sein.

Die Aufteilung des Meßgases in zwei Teilströme kann mittels einstellbarer Durchflußmesser bzw. -regler erfolgen.

Erfindungsgemäß wird weiterhin eine Meßzelle zur Verfügung gestellt, die dadurch gekennzeichnet ist, daß sie zwei Elektrodenwicklungen enthält, die über ihre elektrischen Anschlüsse unabhängig voneinander mit einer Meßspannungsquelle verbindbar sind.

Mit einer derartigen Meßzelle ist es möglich, beide Meßfühler mit Meßgas zu beaufschlagen, jedoch wahlweise nur in einem der Meßfühler die Elektrolyse durchzuführen. Bevor eine Meßzelle sich "erschöpft" bzw. "sättigt" wird dann nach einer gewissen Betriebszeit die andere Meßzelle durch Anlegen der Gleichspannung aktiviert.

Die Elektrodendrähte sind in der Regel Platindrähte. Für normale Anwendungen, d.h. die Messung von Feuchte in nicht aggressiven Gasen wie Luft und dergleichen, ist die Auswahl der Materialien für die Meßzelle nicht besonders kritisch. Als Isolatorkörper wird vorzugsweise ein Glaskörper gewählt, auf den die Elektrodendrähte parallel mit gleichbleibendem Abstand gewickelt werden. Zur besseren Fixierung der Elektrodendrähte ist der Glaskörper mit vorbereiteten Nuten für die Drähte versehen, so daß die Elektrodenwicklung positionsgenau angebracht werden kann und auch gegen Verschieben der Wicklungen gesichert sind. Diese Anordnung wird mit dem Elektrolyten beschichtet, der im Normalfall Phosphorpentoxid ist. Andere Ausführungsformen sind möglich.

Die Länge jedes Elektrodendrahtes liegt in der Größenordnung von einem Meter. Bei bekannten Meßfühlern betrug der Abstand zwischen den Elektrodendrähten in der Wicklung etwa 1 mm. Bei einer solchen Anordnung ist bei erfindungsgemäßen Doppelzellen eine Betriebszeit eines Meßfühlers bis zur Umschaltung auf den anderen von mindestens etwa 10 min. erforderlich.

Es hat sich gezeigt, daß die Ansprechzeit eines Meßfühlers erheblich verbessert werden kann, wenn der Abstand der gegenseitigen Elektrodendrähte verringert wird. Fertigungstechnisch ist es möglich geworden, Meßfühler mit einem Elektrodenabstand von nur etwa 0,1 mm zu fertigen. Diese Ausführung macht es bei der Doppelzelle möglich, mit den Umschaltzeiten bis auf etwa 1 min. herunterzugehen. Da sich somit der Elektrolyt in verhältnismäßig kurzen Zeitabständen erholen kann, steigt die Meßgenauigkeit der Zelle.

Durch diesen Umstand, verbunden mit einer intensiven Umströmung der Meßfühler durch das Meßgas, wie es sich aus der folgenden Beschreibung der Ausführungsbeispiele von erfindungsgemäßen Meßzellen ergibt, verbessert sich die Linearität der Meßcharakteristik im Bereich geringer Gasfeuchte, so daß das Meßverfahren verläßlich auch im ppb-Bereich eingesetzt werden kann.

Vorzugsweise ist die neue Meßzelle auf eine Gesamt-Meßgasdurchflußmenge von 100 N1/h ausgelegt, entsprechend der Auslegung des bekannten Meßgerätes. Bei der neuen Doppelmeßzelle kann an einen der Meßgas-Auslässe oder Eingänge ein Durchflußmengeneinsteller angeschlossen, der auf eine Durchflußmenge von 50 N1/h eingestellt ist. Man erreicht dadurch, daß der Meßgasstrom hälftig auf die beiden Teilkammern aufgeteilt wird.

Im folgenden wird anhand der Figuren der beigefügten Zeichnung nur die erfindungsgemäße Meßzelle im einzelnen näher beschrieben. In der Zeichnung stellen dar:
- Fig. 1: einen schematischen Längsschnitt durch eine erste Ausführungsform einer Meßzelle, und
- Fig. 2: einen schematischen Längsschnitt durch eine andere Ausführungsform einer Meßzelle.

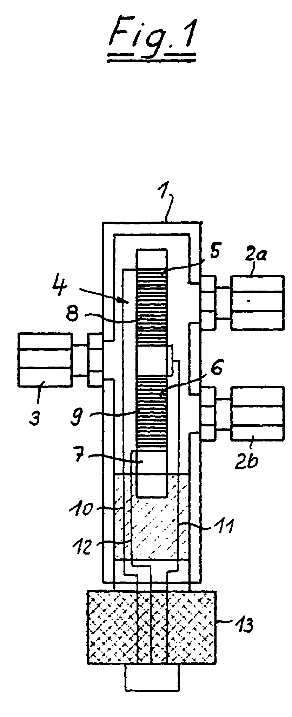

Bei der in Figur 1 dargestellten ersten Ausführungsform ist die Meßfühleranordnung 4 in einem Gehäuse 1 angeordnet, das mit Einlaß- und Auslaßmitteln 2,3 für ein Meßgas ausgestattet ist. Die Meßfühleranordnung 4 umfaßt zwei Meßfühler 5,6, die übereinander auf einem Isolatorkörper 7 angeordnet sind, der auf seiner Oberfläche mit einem hykroskopischen Elektrolytfühler versehen ist. Die Meßfühler 5,6 weisen je eine Elektrodenwicklung 8,9 auf, die paarweise um den Isolatorkörper 7 gewickelt und in den Elektrolytfilm eingebettet sind. Über nach außen geführte elektrische Anschlüsse 10,11,12 können die Elektrodenwicklungen 8,9 unabhängig voneinander mit einer Meßspannungsquelle verbunden werden. Im Hinblick auf die bei längerem Gebrauch beobachtete Erschöpfung bzw. Sättigung werden die beiden Meßfühler 5,6 periodisch abwechselnd mit der Meßspannungsquelle verbunden, so daß jeweils einer des Fühlers 5,6 für die laufenden Messung zur Verfügung steht, während sich der andere in einer Art Regenerationsphase befindet. Das Umschalten erfolgt zweckmäßigerweise, wie bereits erwähnt, mit einer gewissen Überlappungszeit während der die Meßwerte beider Meßfühler 5,6 gemittelt werden.

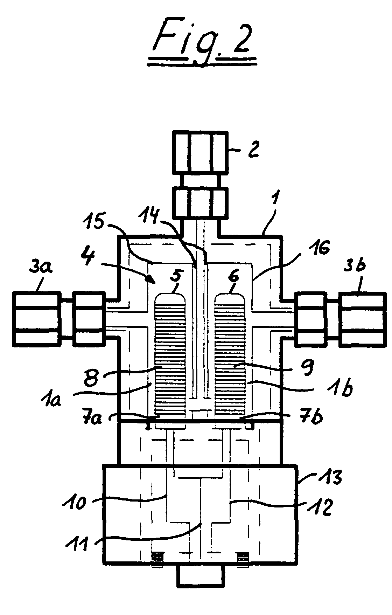

Die Ausführungsform gemäß Figur 2 unterscheidet sich von denjenigen gemäß Figur 1 dadurch, daß am Gehäuse 1 nur ein Einlaßmittel 2 und zwei Auslaßmittel 3a,3b für das Meßgas vorgesehen sind. Die Meßfühler 5,6 sind auf Isolatoren 7a,7b nebeneinander angeordnet und wiederum über elektrische Anschlüsse 10,11,12 mit einer Meßspannungsquelle verbindbar. Die elektrischen Anschlüsse 10,11,12 verlaufen wie bei der Ausführungsform gemäß Figur 1 durch das Gehäuse 1 und ein Sockelteil 13. Die Elektrodenwicklungen sind wiederum mit 8 und 9 bezeichnet. Zur Führung des Gasstroms innerhalb des Gehäuses 1 ist eine Abtrennung 14 vorgesehen, durch die das Gehäuse 1 in zwei Teilkammern 1a, 1b unterteilt wird. Ferner sind Strömungsführungsmittel 15,16 für das eintretende Meßgas vorgesehen, mittels derer das eintretende Meßgas zunächst nach unten und danach von unten nach oben an den Elektrodenwicklungen 8,9 vorbei zu den am Gehäuse 1 diametral gegenüberliegenden Auslaßstutzen 3a, 3b geführt wird.

Selbstverständlich sind weitere Anordnungsmöglichkeiten für die beiden erfindungsgemäß vorgesehenen Elektrodenwicklungen 8,9 denkbar. Maßgebend ist, daß sie von etwa gleich großen Teilströmen des Meßgases beaufschlagt und elektrisch unabhängig voneinander einschaltbar sind. Auf diese Weise kann - wie erwähnt - nicht nur eine dauerhaft exakte Messung durchgeführt werden, es kann auch der Meßbereich erweitert werden.

## Patentansprüche

1. Verfahren zum Messen des Feuchtegehaltes von Gasen, bei dem ein definierter Strom des zu messenden Gases einen Meßfühler mit einem Paar in einen hygroskopischen Elektrolytfilm eingebetteter und mit einer Meßspannung beaufschlagter Elektroden umströmt, und der zwischen den Elektroden fließende Elektrolysestrom als Maß für den Feuchtegehalt des Gases bestimmt wird, dadurch gekennzeichnet, daß mindestens zwei Meßfühler (5, 6) verwendet werden, die in Zeitabständen von beispielsweise 1 bis 10 min. wechselweise mit der Meßspannung beaufschlagt werden.

2. Verfahren nach Anspruch 1, dadurch gekennzeichnet, daß zwei Meßfühler (5, 6) verwendet werden, die in einer gemeinsamen Meßzelle angeordnet sind.

3. Verfahren nach Anspruch 2, dadurch gekennzeichnet, daß der Meßgasstrom von beispielsweise 10 N1/h gleichmäßig auf beide Meßfühler (5, 6) verteilt wird.

4. Gerät zum Messen des Feuchtegehaltes von Gasen mit mindestens einem in einer Meßzelle angeordneten Meßfühler mit einem Isolatorkörper, der an seiner Oberfläche mit einem hygroskopischen Elektrolytfilm versehen ist, und einer Elektrodenwicklung mit einem Paar in den Elektrolytfilm eingebetteter, beabstandeter Elektrodendrähte, und mit einer mit dem mindestens einen Meßfühler elektrisch verbindbaren Versorgungs- und Erfassungseinheit mit Mitteln zum Beschalten der Elektrodenwicklung des mindestens einen Meßfühlers mit einer Meßspannung und Mitteln zum Messen des Stromflusses durch den Meßfühler und zum Umwandeln des Stromflußwertes in ein Ausgabesignal für den Feuchtegehalt des Gases, dadurch gekennzeichnet, daß das Gerät mindestens zwei gleichzeitig mit der Versorgungs- und Erfassungseinheit verbindbare Meßfühler (5, 6) aufweist und die Versorgungs- und Erfassungseinheit mit Schaltmitteln zum im wesentlichen wechselweisen automatischen Umschalten von einem aktuellen Meßfühler (5, 6) auf einen anderen aktuellen Meßfühler (5, 6) in insbesondere vorwählbaren Zeitintervallen versehen ist.

5. Gerät nach Anspruch 4, gekennzeichnet durch Mittel zum Halten des Ausgabewertes des zuletzt in Betrieb gewesenen Meßfühlers und zum Vergleich dieses Wertes mit dem Ausgabewert des aktuellen Meßfühlers, zumindest für ein insbesondere wählbares Zeitintervall.

6. Gerät nach Anspruch 5, dadurch gekennzeichnet, daß die Meßfühler (5, 6) zeitlich überlappend umschaltbar sind.

7. Gerät nach Anspruch 5 oder 6, dadurch gekennzeichnet, daß Mittel zum Erzeugen eines Alarms vorgesehen isnd, die auslösbar sind, wenn der Ausgabewert des aktuellen Meßfühlers eine bestimmte Abweichung vom letzten Ausgabewert des zuletzt aktuellen Meßfühlers aufweist.

8. Gerät nach mindestens einem der Ansprüche 4 bis 7, dadurch gekennzeichnet, daß es zwei Meßfühler (5, 6) aufweist, die in einer gemeinsamen Meßzelle angeordnet sind.

9. Meßzelle für ein Gerät zum Messen des Feuchtegehaltes von Gasen mit e9inem von dem zu messenden Gas durchströmbaren Gehäuse (1) mit Einlaß- und Auslaßmitteln (2,3) für das Meßgas und einer in dem Gehäuse (1) vorgesehenen Meßfühleranordnung (4) mit mindestens einem Meßfühler (5,6) mit einem Isolatorkörper, der an seiner Oberfläche mit einem hykroskopischen Elektrolytfilm versehen ist, und mindestens einer Elektrodenwicklung (8,9) mit einem Paar um den Isolatorkörper (7) gewickelter und in den Elektrolytfilm eingebetteter, beabstandeter Elektrodendrähte, die mit durch das Gehäuse (1) nach außen geführten elektrischen Anschlüssen (10,11,12) versehen sind, für das Anlegen einer Meßspannung an die Elektrodenwicklungen (8,9),
dadurch gekennzeichnet, daß zwei Meßfühler (5, 6) vorgesehen sind, die über ihre elektrischen Anschlüsse (10,11,12) unabhängig voneinander mit einer Meßspannungsquelle verbindbar sind.

10. Meßzelle nach Anspruch 9, dadurch gekennzeichnet, daß die beiden Meßfühler (5, 6) einen gemeinsamen Isolatorkörper (7) aufweisen und die Elektrodenwicklungen (8,9) versetzt zueinander auf dem Isolatorkörper (7) angeordnet sind.

11. Meßzelle nach Anspruch 1, dadurch gekennzeichnet, daß die beiden Elektrodenwicklungen (8,9) auf getrennten Isolatorkörpern (7a,7b) angeordnet sind, die je in einer Teilkammer (1a, 1b) des Gehäuses (1) angeordnet sind.

12. Meßzelle nach mindestens einem der Ansprüche 9-11, dadurch gekennzeichnet, daß sie mit Mitteln zum gleichmäßigen Aufteilen des Meßgasstromes auf die beiden Elektrodenwicklungen (8, 9) versehen bzw. verbindbar ist.
